Europäisches Patentamt

European Patent Office

Office européen des brevets

(11) Veröffentlichungsnummer: **0 352 415**
**A2**

## (12) EUROPÄISCHE PATENTANMELDUNG

(21) Anmeldenummer: **89108182.0**

(51) Int. Cl.⁴: **A61M 16/08**

(22) Anmeldetag: **05.05.89**

(30) Priorität: **23.07.88 DE 8809453 U**

(43) Veröffentlichungstag der Anmeldung:
**31.01.90 Patentblatt 90/05**

(84) Benannte Vertragsstaaten:
**AT DE FR IT**

(71) Anmelder: **WILLY RÜSCH AG**

**D-7050 Waiblingen(DE)**

(72) Erfinder: **Rüsch, Heinz Dipl.-Ing.**
**Nachtigallenweg 6**
**D-7050 Waiblingen(DE)**

(74) Vertreter: **Patentanwälte Kohler - Schwindling**
**- Späth**
**Hohentwielstrasse 41**
**D-7000 Stuttgart 1(DE)**

(54) **Tracheostomie-Set.**

(57) Ein Tracheostomie-Set weist einen zweiten und dritten Rohrabschnitt 24, 25 auf. Der zweite und der dritte Rohrabschnitt 24, 25 sind unmittelbar im Anschluß an den mit einem Konnektor 12 eines Tracheostomie-Tubus in Eingriff bringbaren Verbindungsteil eines ersten Rohrabschnittes 21 angeordnet. Die Anordnung erfolgt im wesentlichen in der gleichen Querschnittsebene und im wesentlichen rechtwinklig zur Achse des ersten Rohrabschnittes 21. Der zweite und der dritte Rohrabschnittt 24, 25 münden in die Wandung des ersten Rohrabschnittes 21 und der erste Rohrabschnitt 21 ist an seinem freien Ende mit einer Endwand 23 versehen. Die Anordnung der Rohrabschnitte erlaubt auch in der Bauchlage eine problemlose Beatmung von Kleinkindern.

Fig.1

### Tracheostomie-Set

Die Erfindung betrifft ein Tracheostomie-Set, bestehend aus einem Tracheostomie-Tubus und einem Winkelstück zur Herstellung der Verbindung zwischen dem am distalen Ende des Tracheostomie-Tubus angebrachten Konnektor und den Schläuchen eines Beatmungsgeräts, welches Winkelstück an einem Ende einen mit dem Konnektor des Tracheostomie-Tubus verbindbaren ersten Rohrabschnitt aufweist, an den sich ein zweiter und ein dritter Rohrabschnittt mit voneinander entfernten Enden anschließen, von denen jeder an seinem Ende mit einem Standard-Konnektor zum Anschluß eines der Schläuche des Beatmungsgerätes versehen ist.

Wenn Patienten, die einen Tracheostomie-Tubus tragen, eine Narkose erhalten sollen, ist es erforderlich, an das distale Ende des Tracheostomie-Tubus ein Beatmungsgerät anzuschließen. Daher ist es erforderlich, zwischen dem Tracheostomie-Tubus und den Schläuchen des Beatmungsgerätes ein Zwischenstück anzuordnen, das einen ersten Rohrabschnitt aufweist, der mit dem distalen Ende des Tracheostommie-Tubus verbindbar ist, und das sich in zwei weitere Rohrabschnitte verzweigt, an die dan die beiden Schläuche des Beatmungsgerätes anschließbar sind. Bei Narkosegeräten haben sich allgemein genormte Standard-Kon-nektoren durchgesetzt, mit denen auch die Enden des Zwischenstückes versehen sind, und zwar gewöhnlich auch das Ende des ersten Rohrabschnittes, das mit dem Ende des Tracheostomie-Tubus zu verbinden ist. Da diese Standard-Konnektoren einen erheblichen Durchmesser und eine erhebliche Länge haben, so daß sie am Ende des Tracheostomie-Tubus stören würden, ist gewöhnlich ein weiteres Zwischenstück erforderlich, das an einem Ende einen an den Konnektor des Tracheostomie-Tubus angepaßten Konnektor und am anderen Ende einen Standard-Konnektor aufweist. Damit die gesamte Anordnung keinen zu weit vom Hals des Patienten abstehenden Aufbau ergibt, ist das sich verzweigende Zwischenstück gewöhnlich als Winkelstück ausgebildet, und zwar in der Weise, daß der erste Rohrabschnitt rechtwinklig abgebogen ist und sich die beiden weiteren Rohrabschnitte an den abgebogenen Teil des ersten Rohrabschnittes anschließen, indem sie sich entweder Y-förmig verzweigen (Y-Konnektor) oder aber mit Abstand voneinander seitlich an den abgebogenen Teil des ersten Rohrabschnittes anschließen (M-Konnektor).

Ein wesentlicher Nachteil dieser Anordnungen besteht darin, daß in dem Bereich zwischen dem Anschluß an den Tracheostomie-Tubus und der Stelle, an der sich die beiden, zum Zuführen der einzuatmenden bzw. Abführen der ausgeatmeten Luft dienenden Luftwege an der Verzweigung des ersten Rohrabschnittes vereinigen, ein erheblicher Abstand besteht, also der erste Rohrabschnitt des Winkelstückes gegebenenfalls in Verbindung mit dem weiteren Zwischenstück ein erhebliches Luftvolumen einschließt, das als sogenannter "Totraum" bei der Atmung nicht in die Gasaustauschzone der Lunge gelangt. Auch wenn dieser Totraum bei erwachsenen Patienten im allgemeinen tolerierbar ist, kann es in kritischen Situationen doch zu Komplikationen führen. Bei Kindern, und insbesondere Kleinkindern, stellt dieser Totraum stets ein ernsthaftes Problem und ein echtes Gefahrenpotential dar.

Ein weiterer Nachteil der bekannten Anordnungen besteht in ihrem sperrigen Aufbau, der trotz Verwendung eines Winkelstückes den Anschluß der Schläuche des Beatmungsgerätes erst in einem erheblichen radialen Abstand vom Hals des Patienten gestattet. Dieser große Abstand ist besonders dann störend, wenn ein Patient in Bauchlage behandelt werden muß, so daß der Kopf in einem ausreichend großen Abstand vom Operationstisch gehalten werden muß, um das Winkelstück und gegebenenfalls ein weiteres Zwischenstück zwischen dem Hals des Patienten und der Oberfläche einer Liege, insbesondere eines Operationstisches, unterbringen zu können. Auch hier wird dieses Problem um so größer, je kleiner der Patient ist, so daß bei der Behandlung von Kleinkindern erhebliche Probleme für die Lagerung des Patienten entstehen.

Der Erfindung liegt die Aufgabe zugrunde, ein Tracheostomie-Set zu schaffen, bei dem der Totraum auf ein Minimum reduziert ist und dessen Aufbau auch bei einer Bauchlage des Patienten einen problemlosen Anschluß der Schläuche des Beatmungsgerätes ermöglicht.

Diese Aufgabe wird nach der Erfindung dadurch gelöst, daß der zweite und der dritte Rohrabschnitt unmittelbar im Anschluß an den mit dem Konnektor des Tracheostomie-Tubus in eingriff bringbaren Verbindungsteil des ersten Rohrabschnittes im wesentlichen in der gleichen Querschnittsebene und im wesentlichen rechtwinklig zur Achse des ersten Rohrabschnittes in dessen Wandung münden und der erste Rohrabschnitt an seinem dem Verbindungteil abgewandten Ende durch eine den Mündungen der zweiten und dritten Rohrabschnitte benachbarte Endwand abgeschlossen ist.

Bei dem erfindungsgemäßen Tracheostomie-Set ist demgemäß der Totraum auf einen extrem kurzen Rohrabschnitt beschränkt, dessen Länge im wesentlichen gleich dem Durchmesser der sich radial anschließenden zweiten und dritten Rohrabschnitte ist. Ebenso ist auch die radiale Ausdehnung der Anordnung vom Hals des Patienten nur um den Durchmesser dieser Rohrabschnitte vergrößert, da diese Rohrabschnitte unmittelbar an das distale Ende des Tracheostomie-Tubus angrenzen. Daher erlaubt dieser Tracheostomie-Set eine völlig problemlose Beatmung, selbst bei Kleinstkindern, und zwar auch in der Bauchlage, weil die zum Hals radiale Ausdehnung der Anordnung normalerweise nicht größer ist als der Überstand des Kinns über die Außenfläche des Halses.

Bei einer bevorzugten Ausführungsform der Erfindung verlaufen die an den ersten Rohrabschnitt anschließenden Teil des zweiten und des dritten Rohrabschnittes parallel zueinander. Auf diese Weise lassen sich die Anschlüsse für die Schläuche des Beatmungsgerätes an der gleichen Seite des Körpers anordnen, und es liegen dort die Anschlüsse für die Schläuche des Beatmungsgerätes im wesentlichen nebeneinander. Dabei besteht sowohl die Möglichkeit, die zweiten und dritten Rohrabschnitte als selbständige, wenn auch vorzugsweise miteinander verbundene Rohr- oder Schlauchabschnitte auszubilden, als auch ein einstückiges Rohr mit einer zentralen Scheidewand zu verwenden. Dabei können die mit den Standard-Konnektoren zum Anschluß des Beatmungsgerätes versehenen Enden der zweiten und dritten Rohrabschnitte Y-förmig auseinanderstreben. Damit diese Standard-Konnektoren für den Anschluß der Schläuche des Beatmungsgerätes gut zugänglich sind, sollten sie neben dem Hals des Patienten frei liegen, ohne jedoch zu weit überzustehen, damit sie auf der den Patienten aufnehmenden Liege noch aufliegen. Deshalb sollte die Länge der zweiten und dritten Rohrabschnitte von dem ersten Rohrabschnitt bis zu den Standard-Konnektoren 8 bis 16 cm und vorzugsweise etwa 12 cm betragen.

Ein weiterer Vorteil der erfindungsgemäßen Ausbildung des Tracheostommie-Sets besteht darin, daß die den ersten Rohrabschnitt abschließende Endwand senkrecht zur Achse des distalen Endes des Tracheostomie-Tubus angeordnet ist, so daß sie mit einer Öffnung versehen werden kann, von der aus der Tracheostomie-Tubus zugänglich ist, beispielsweise zum Einführen eines Absaug-Katheders oder der Fiberoptik eines Endoskops. Daher sieht eine bevorzugte Ausführungsform der Erfindung vor, daß die Endwand des ersten Rohrabschnittes eine zentrale, durch einen lösbaren Stopfen verschlossene Öffnung aufweist.

Um eine besonders sichere Verbindung des Winkelstückes mit dem Trachstomie-Tubus zu gewährleisten, kann in weiterer Ausgestaltung der Erfindung wenigstens der erste Rohrabschnitt aus einem weichen Kunststoff bestehen und am Ende seines Verbindungsteiles einen nach innen vorspringenden Bund aufweisen, der in eine entsprechende Ringnut am Umfang des am Tracheostomie-Tubus angebrachten Konnektors eingreift. Auf diese Weise wird eine formschlüssige Verbindung erzielt, die auch bei sehr kleinen Abmessungen des Verbindungsteiles eine sehr sichere Verbindung zwischen dem Winkelstück und dem Tracheostomie-Tubus geewährleistet.

Die erfindungsgemäße Ausbildung des Tracheostomie-Set bietet auch die Möglichkeit, das gesamte Winkelstück aus einem weichen Kunststoff herzustellen, weil es keinen sperrigen Aufbau bildet, sondern so angeordnet werden kann, daß auch seine zweiten und dritten Rohrabschnitte auf einer Unterlage aufliegen. Dabei gewährleistet die Nachgiebigkeit eines weichen Kunststoffes, daß unzulässige Kräfte von dem Tracheostomie-Tubus und daher auch von dem sehr empfindlichen Stoma-Bereich des Patienten ferngehalten werden.

Die Verwendung eines weichen Kunststoffes hat bei einem Tracheostomie-Set, bei dem die Endwand des festen Rohrabschnittes eine Öffnung aufweist, den weiteren Vorteil, daß diese Öffnung einen etwas kleineren Durchmesser haben kann als die dadurch einzuführenden Instrumente, so daß sich der Rand der Öffnung an den Umfang des eingeführten Instrumentes zugleich haltend und dichtend anlegt. Die Nachgiebigkeit der Endwand kann dann noch dadurch verbessert werden, daß die Dicke der Endwand in der Umgebung der Öffnung vermindert ist.

Weiterhin sollte das Winkelstück aus einem wenigstens durchscheinenden und vorzugsweise durchsichtigen Kunststoff bestehen, damit das Auftreten von Ausscheidungen aus der Trachea beobachtet werden kann.

Bei dem erfindungsgemäßen Tracheostomie-Set besteht keine Notwendigkeit, am distalen Ende des Tracheostomie-Tubus einen Standard-Konnektor anzuordnen, weil hier keine Fremdgeräte anzuschließen sind, sondern lediglich zum Tracheostomie-Set gehörende Teile, insbesondere das nach der Erfindung ausgebildete Winkelstück. Daher besteht die Mögllichkeit, den am distalen Ende des Tracheostomie-Tubus angebrachten Konnektor für den Anschluß solcher Teile optimal auszubilden. Insbesondere kann auch der Konnektor selbst im Hinblick auf den angestrebten kleinen Totraum und eine geringe räumliche Ausdehnung möglichst klein gehalten werden. Die oben erwähnte Ausbildung des ersten Rohrabschnittes aus einem weichen Kunststoff, in Verbindung mit dem in eine Ringnut am Umfang des Konnektors eingreifenden Bund ermöglicht eine sehr kleine Ausbildung des Konnektors bei gleichzeitiger Herstellung einer sehr sicheren Verbindung.

Ebenso wie das Winkelstück könnten auch andere anzuschließende Teile ein nachgiebiges Verbindungteil aufweisen, das an seinem Ende einen nach innen vorspringenden Bund aufweist. Für manche Zusatzgeräte, wie beispielsweise eine Hustenkappe, eine Verschlußkappe und dergleichen kann es jedoch erwünscht sein, eine einfachere Anschlußmöglichkeit zu haben. Daher ist in weiterer Ausgestaltung der Erfindung vorgesehen, daß der Konnektor des Tracheostomie-Tubus an seinem Umfang mit Verriegelungsmitteln für solche Zusatzeinrichtungen versehen ist. Dabei kann es sich insbesondere um Verriegelungsmittel handeln, die nach Art eines Bajonett-Verschlusses wirken. Bei einer bevorzugten Ausführungsform der Erfindung werden die Verriegelungsmittel von Vorsprüngen gebildet, welche die Gänge eines 4-gängigen Steilgewindes definieren. Ein solches Steilgewinde ermöglicht eine sichere Befestigung von Zusatzeinrichtungen durch eine einfache, kurze Drehbewegung und das Aufsetzen der Zusatzeinrichtungen in vier unterschiedlichen Winkelstellungen.

Die Erfindung wird im folgenden anhand des in der Zeichnung dargestellten Ausführungsbeispieles näher beschrieben und erläutert. Die der Beschreibung und der Zeichnung zu entnahmenden Merkmale können bei anderen Ausführungsformen der Erfindung einzeln für sich oder zu mehreren in beliebiger Kombination Anwendung finden. Es zeigen

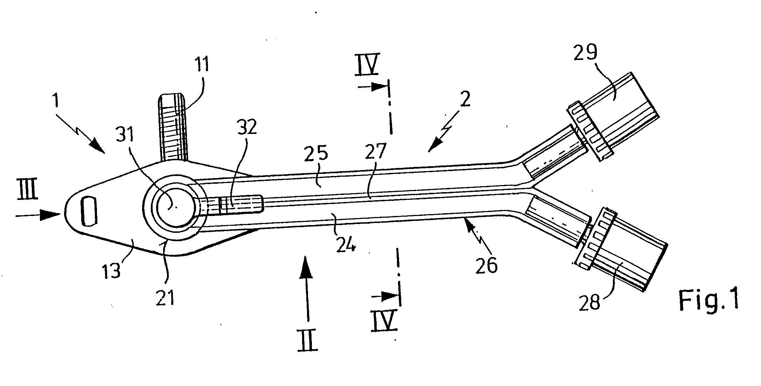

Fig. 1 eine Draufsicht auf ein Tracheostomie-Set nach der Erfindung,

Fig. 2 eine Ansicht des Tracheostomie-Set nach Fig. 1 in Richtung des Pfeiles II,

Fig. 3 eine Ansicht des Tracheostomie-Set nach Fig. 1 in Richtung des Pfeiles III und

Fig. 4 einen Schnitt längs der Linie IV-IV durch das Winkelstück des Tracheostomie-Sets nach Fig. 1.

Das in der Zeichnung dargestellte Tracheostomie-Set umfaßt einen Tracheostomie-Tubus 1 und ein damit lösbar verbundenes Winkelstück 2.

Der Tracheostomie-Tubus 1 besteht aus einem gebogenen Rohrstück 11 aus weichem Kunststoff, das durch eine eingelegte Metallspirale verstärkt ist. An einem Ende des Rohrstückes 11 ist ein Konnektor 12 befestigt, bei dem es sich um ein hülsenförmiges Kunststoffteil handelt, in dessen Bohrung das Ende des Rohrstückes 11 befestigt ist. Nahe seinem in Bezug auf das äußere oder distale Ende des Tracheostomie-Tubus hinteren Ende weist der Konnektor 12 eine Ringnut auf, in die der Rand einer zentralen Öffnung einer Halteplatte 13 eingreift. Zur Halteplatte 13 benachbart ist am Umfang des Konnektors 12 eine Ringnut 14 angeordnet, während sich an der daran anschließenden Umfangsfläche des Konnektors 12 Vorsprünge 15 befinden, welche die Gänge eines 4-gängigen Steilgewindes definieren.

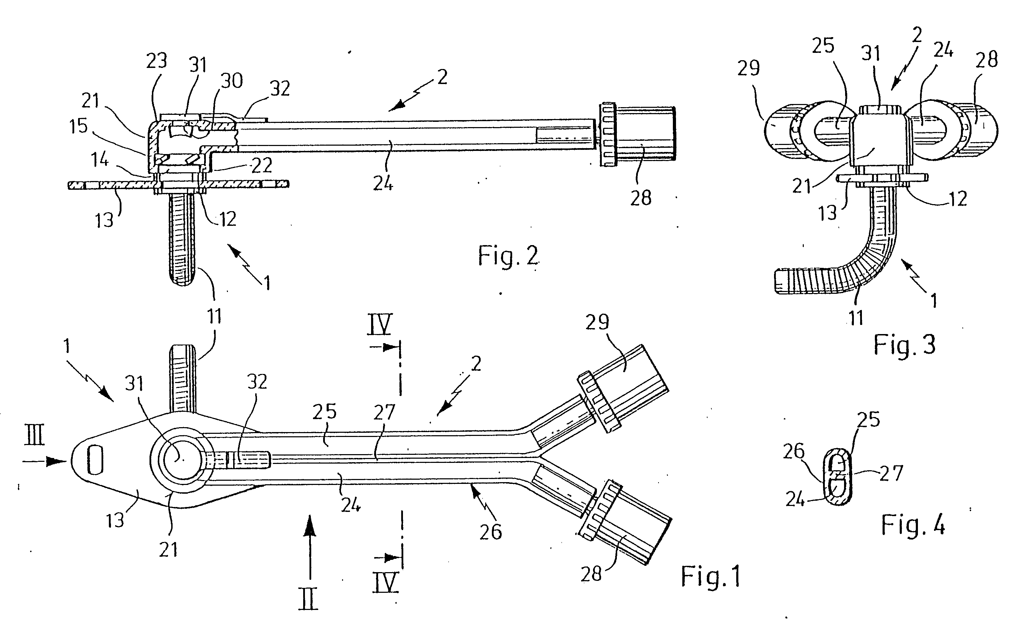

Das Winkelstück 2 umfaßt einen ersten, kurzen Rohrabschnitt 21, der an einem Ende einen nach innen vorspringenden Bund 22 aufweist und am anderen Ende durch eine Endwand 23 verschlossen ist. Dieser erste Rohrabschnitt 21 umgibt den Konnektor 12 des Tracheostomie-Tubus 1 und greift mit seinem Bund 22 in dessen Ringnut 14 ein. Die Länge des ersten Rohrabschnittes 21 ist so bemessen, daß dieser Rohrabschnitt über den Konnektor 12 des Tracheostomie-Tubus 1 nur so weit übersteht, wie es der Durchmesser von sich radial an der ersten Rohrabschnitt anschließenden zweiten und dritten Rohrabschnitten 24 und 25 erfordert. Diese zweiten und dritten Rohrabschnitte 24, 25 verlaufen parallel zueinander und werden von einem Kunststoffschlauch 26 gebildet, der einen langrunden Querschnitt hat und zwei durch eine Scheidewand 27 getrennte Lumen aufweist (siehe Fig. 4). Die von dem ersten Rohrabschnitt 21 abgewandten Enden der zweiten und dritten Rohrabschnitte 24, 25 streben Y-förmig auseinander und sind an ihren Enden mit Standard-Konnektoren 28, 29 versehen, die in nicht näher dargestellter Weise den Anschluß der Schläuche eines Beatmungsgerätes gestatten. Dabei dienen von den zweiten und den dritten Rohrabschnitten der eine der Inspiration und der andere der Expiration.

Die Endwand 23 des ersten Rohrabschnittes 21 ist mit einer zentralen Öffnung 30 versehen, die normalerweise durch einen Stopfen 31 verschlossen ist. Dieser Stopfen ist durch ein flexibles Band 32 mit der Außenseite des Winkelstückes, bei dem dargestellten Ausführungsbeispiel mit der Außenseite des den zweiten und den dritten Rohrabschnitt bildenden Kunststoffschlauches 26, verbunden. In einem die Öffnung 30 umgebenden Bereich 33 ist die Dicke der den ersten Rohrabschnitt 21 abschließenden Endwand 23 vermindert.

Bei dem dargestellten Ausführungsbeispiel besteht das Winkelstück 2, abgesehen von den Standard-Konnektoren 28, 29, aus einem flexiblen Kunststoff, so daß der erste Rohrabschnitt leicht auf den am distalen Ende des Tracheostomie-Tubus angeordneten Konnektor 12 aufgesetzt und auch wieder von diesem Konnektor gelöst werden kann. Dabei gewährleistet der am Ende des ersten Rohrabschnittes 21 angebrachte Bund 22 durch seinen formschlüssigen Eingriff in die Ringnut 14 am Umfang des Konnektors 12 stets eine sichere Verbindung zwischen dem Winkelstück und dem Tracheostomie-Tubus 1. Wegen der geringen Länge des ersten Rohrabschnittes 21 und des Anschlusses der zweiten und dritten Rohrabschnitte unmittelbar an den Umfang des ersten Rohrschnittes wird der Totraum der Anordnung, das heißt das Luftvolumen, in dem die Inspirations-Luft und die Expirations-Luft nicht mehr durch den zweiten und den dritten Rohrabschnitt getrennt sind, auf ein Minimum reduziert. Weiterhin verlaufen der zweite und der dritte Rohrabschnitt 24, 25 des Winkelstückes 2 in einem sehr geringen Abstand von der Halteplatte 13 parallel zu dieser Halteplatte und infolgedessen, bei eingesetzten Tracheostomie-Tubus, im wesentlichen tangential zum Hals des den Tracheostomie-Tubus tragenden Patienten, so daß die Anordnung auch dann unterhalb des Kinnes eines Patienten ausreichend Platz hat, wenn der Patient in Bauchlage behandelt werden muß. Dabei kann die Größe des Tracheostomie-Sets der Körpergröße des Patienten angepaßt werden. Insbesondere können solche Tracheostomie-Sets in entsprechend kleinen Größen für Kinder und selbst Kleinstkinder ausgebildet werden, die somit mühelos behandelt werden können. Gerade für die Behandlung von Kleinstkindern ist auch die Reduzierung des Totraums auf ein absolut unvermeidbares Minimum von großer Bedeutung.

Weiterhin kann die Länge des Kunststoffschlauches 26 mit den zweiten und dritten Rohrabschnitten 24, 25 so bemessen werden, daß die Standard-Konnektoren 28, 29 zum Anschluß der Schläuche der Beatmungsgeräte neben dem Hals des Patienten frei zugänglich sind. Je nach der Größe des Patienten kann daher die Länge dieses Kunststoffschlauches 26, gemessen von dem ersten Rohrabschnitt 21 bis zu den Konnektoren 28, 29, 8 bis 16 cm betragen. Ein für viele Zwecke geeignetes Maß für diese Länge ist 12 cm.

Die Verwendung eines flexiblen Kunststoffes hat weiterhin den Vorteil, daß die beim Anschluß der Schläuche und die im Betrieb auf die Konnektoren ausgeübten Kräfte viel besser abgefangen werden können und daher nicht so unmittelbar auf den Patienten übertragen werden wie bei starrer Ausbildung von Winkelstück und Tracheostomie-Tubus.

Für die Beobachtung des Beatmungs-Vorganges und insbesondere für die Beobachtung von während der Beatmung auftretenden Ausscheidungen ist es vorteilhaft, wenn das Winkelstück aus einem durchsichtigen oder wenigstens durchscheinenden Kunststoff besteht. Wenn Ausscheidungen beobachtet werden, erlaubt es die bei dem dargestellten Auführungsbeispiel in der Endwand 23 des ersten Rohrabschnittes 21 angeordnete Öffnung 30, die sich in Verlängerung der Achse des Rohrstükkes 11 des Tracheostomie-Tubus befindet, das Einführen eines Absauge-Katheters. Auch hierfür ist es von Vorteil, wenn das Winkelstück aus einem weichen, das heißt elastisch nachgiebigen Kunststoff besteht, weil dann durch die Öffnung 30 auch Absauge-Katheter und andere Instrumente eingeführt werden können, deren Durchmesser größer ist als der Durchmesser der Öffnung 30. Die Öffnung wird dann entsprechend aufgeweitet, und es liegt der Rand der Öffnung dicht an den Umfang des eingeführten Instrumentes an. Daher können Manipulationen mit einem solchen Instrumen vorgenommen werden, ohne daß dabei infolge des Eintrittes von Nebenluft der Beatmungsvorgang gestört wird. Durch die Verminderung der Dicke der Endwand 23 in dem die Öffnung 30 umgebenden Bereich wird das Einführen dickerer Instrumente erleichtert und ein gutes Anschmiegen des die Öffnung 30 begrenzenden Randes der Endwand 23 an ein solches Instrument begünstigt. Neben dem Einführen eines Absauge-Katheters gestattet die Öffnung beispielsweise auch die Verwendung einer Fiberoptik. Nach dem Abschluß von Maßnahmen, die das Einführen eines Instrumentes durch die Öffnung 30 erforderlich machen, kann diese Öffnung durch den Stopfen 31 wieder verschlossen werden, der mittels des Bandes 32 mit dem Winkelstück unlösbar verbunden ist.

Es versteht sich, daß die Erfindung nicht auf das dargestellte Ausführungsbeispiel beschränkt ist, sondern Abweichungen davon möglich sind, ohne den Rahmen der Erfindung zu verlassen. So können insbesondere die zweiten und dritten Rohrabschnitte von voneinander getrennten Rohren oder Schläuchen gebildet werden, deren Enden dann bei biegsamer Ausbildung auch nicht abgewinkelt zu werden brauchen, um einen für den Anschluß von Schläuchen ausreichenden Abstand zwischen den Standard-Konnektoren herzustellen. Es wäre auch möglich, die zweiten und dritten Rohrabschnitte unter verschiedenen Winkeln an den ersten Rohrabschitt anzuschließen. Weiterhin bestehen viele Möglichkeiten bezüglich der Werkstoff-Auswahl sowie auch der Ausbildung der Verbindung zwischen dem Tracheostomie-Tubus und dem Winkelstück. Die dargestellte Verbindung mittels eines in eine Umfangsnut am Konnektor des Tracheostomie-Tubus eingreifenden. Bundes am Ende des elastischen ersten Rohrabschittes bietet eine besonders einfach zu handhabende und zugleich sichere Art der Verbindung. Doch könnten auch übliche Steckverbindungen mit Kegeln oder Verriegelungsgliedern Anwendung finden. Beispielsweise könnte zum Anschluß des Winkelstükkes auch die ein viergängiges Gewinde bildenden Vorsprünge 15 am Umfang des Konnektors 12 des Tracheostomie-Tubus Verwendung finden. Bei dem dargestellten Ausführungsbeispiel haben diese Vorsprünge allerdings nur den Zweck, den Anschluß anderer Einrichtungen zu ermöglichen, wie insbesondere das Aufsetzen einer Umlenkkappe (Hustenkappe) oder auch einer Verschlußkappe, wenn geprüft werden soll, ob der Patient die Fähigkeit zur Atmung über die normalen Luftwege wiedererlangt hat oder nicht.

## Ansprüche

1. Tracheostomie-Set, bestehend aus einem Tracheostomie-Tubus und einem Winkelstück zur Herstellung der Verbindung zwischen dem am distalen Ende des Tracheostomie-Tubus angebrachten Konnektor und den Schläuchen eines Beatmungsgeräts, welches Winkelstück an einem Ende einen mit dem Konnektor des Tracheostomie-Tubus verbindbaren ersten Rohrabschnitt aufweist, an den sich ein zweiter und ein dritter Rohrabschnittt mit voneinander entfernten Enden anschließen, von denen jeder an seinem Ende mit einem Standard-Konnektor zum Anschluß eines der Schläuche des Beatmungsgerätes versehen ist, dadurch gekennzeichnet, daß der zweite und der dritte Rohrabschnitt (24, 25) unmittelbar im Anschluß an den mit dem Konnektor (12) des Tracheostomie-Tubus (1) in Eingriff bringbaren Verbindungsteil des ersten Rohrabschnittes (21) im wesentlichen in der gleichen Querschnittsebene und im wesentlichen rechtwinklig zur Achse des ersten Rohrabschnittes (21) in dessen Wandung münden und der erste Rohrabschnitt (21) an seinem zum Verbindungsteil abgewandten Ende durch eine den Mündungen der zweiten und dritten Rohrabschnitte (24,25) benachbarte Endwand (23) abgeschlossen ist.

2. Tracheostomie-Set nach Anspruch 1, dadurch gekennzeichnet, daß die an den ersten Rohrabschnitt (21) anschließenden Teile des zweiten und des dritten Rohrabschnittes (24, 25) parallel zueinander verlaufen.

3. Tracheostomie-Set nach Anspruch 2, dadurch gekennzeichnet, daß die Standard-Konnektoren (28, 29) an Y-förmig auseinanderstrebenden Enden der zweiten und dritten Rohrabschnitte (24, 25) angebracht sind.

4. Tracheostomie-Set nach einem der vorhergehenden Ansprüche, dadurch gekennzeichnet, daß die Länge der zweiten und dritten Rohrabschnitte (24, 25) von dem ersten Rohrabschnitt (21) bis zu den Standard-Konnektoren (28, 29) 8 bis 16 cm und vorzugsweise etwa 12 cm beträgt.

5. Tracheostomie-Set nach einem der vorhergehenden Ansprüche, dadurch gekennzeichnet, daß die Endwand (23) des ersten Rohrabschnittes (21) eine zentrale, durch einen lösbaren Stopfen (31) verschlossene Öffnung (30) aufweist.

6. Tracheostomie-Set nach einem der vorhergehenden Ansprüche, dadurch gekennzeichnet, daß wenigstens der erste Rohrabschnitt (21) aus einem weichen Kunststoff besteht und am Ende seines Verbindungsteiles einen nach innen vorspringenden Bund (22) aufweist, der in eine entsprechende Ringnut (14) am Umfang des am Tracheostomie-Tubus (1) angebrachten Konnektors (12) eingreift.

7. Tracheostomie-Set nach den Ansprüchen 5 und 6, dadurch gekennzeichnet, daß die Dicke der Endwand (23) in der Umgebung der Öffnung (30) vermindert ist.

8. Tracheostomie-Set nach einem der vorhergehenden Ansprüche, dadurch gekennzeichnet, daß das Winkelstück (2) aus einem wenigstens durchscheinenden und vorzugsweise durchsichtigen Kunststoff besteht.

9. Tracheostomie-Set nach einem der vorhergehenden Ansprüche, dadurch gekennzeichnet, daß der Konnektor (12) des Tracheostomie-tubus (1) an seinem Umfang mit Verriegelungsmitteln (15) für Zusatzeinrichtungen versehen ist.

10. Tracheostomie-Set nach Anspruch 9, dadurch gekennzeichnet, daß die Verriegelungsmittel von Vorsprüngen (15) gebildet werden, welche die Gänge eines mehrgängigen Steilgewindes definieren.

Fig.1

Fig. 3

Fig. 4

Fig. 2

Fig. 1

Gm 12 18